# EUROPEAN PATENT APPLICATION

(11) **EP 4 242 251 A1**
(43) Date of publication of application: **13.09.2023**
(21) Application number: 22161692.3
(22) Date of filing: 11.03.2022
(51) Int. Cl.: C08G 75/14, C07C 319/22, C08G 75/16, C08L 81/04

(54) **PRODUCTION OF BIS-THIOL COMPOUNDS AND USE THEREOF IN THE PRODUCTION OF LIQUID POLYSULFIDE POLYMERS**

(71) Applicant: Nouryon Chemicals International B.V., 6824 BM Arnhem (NL)
(72) Inventor: LUNGU, Daniela, 6824BM Arnhem (NL); MENZEL, Manfred, 6824BM Arnhem (NL); KLOBES, Olaf, 6824BM Arnhem (NL)
(74) Representative: LKGlobal UK Ltd.

(57) **Abstract**

The present invention relates to the production of bis-thiol compounds, preferably DMDH/DMDH oligomer mixtures, and their use in the production of liquid polysulfide polymers

## Description

The present invention relates to the production of bis-thiol compounds, preferably 1,7-dimercapto-3,5-dioxaheptane (DMDH) and its oligomer mixtures, and their use in the production of liquid polysulfide polymers

### Background

Polysulfide polymers (mercapto-terminated polymers with the formula HS-R-(S_{y} -R)ₜ - SH where R indicates the polyether backbone, *y* the number of sulfur atoms and *t* the number of repeating units) are produced by a polycondensation of a halide mixture (e.g. a mixture of Bis-(2-chloroethoxy)-methane and 1,2,3-Trichloropropane) and sodium polysulfide (Na₂Sₓ) which leads to a high molecular weight polysulfide latex. This latex is split afterwards to the desired chain length (reductive splitting of disulfide groups in the polymer backbone of the latex) to thereby obtain a liquid polymer (instead of a solid).

Commonly used splitting agents (reducing agents) are sodium dithionite (Na₂S₂O₄) or sodium hydrosulfide/sodium sulfite (NaSH and Na₂SO₃); see, for example, WO2015/128270. The disadvantages of using such splitting agents include the generation of substantial salt waste (wastewater containing sulfates and sulfites), which requires a separate washing step, and that the splitting agent will not be incorporated in the polymer chain (poor atom efficiency).

US 6939941 describes a single step process for making liquid polysulfides that does not require a splitting step. NaSH is reacted with sulfur to form Na₂Sₓ which is then reacted with CI-R-CI. A substantial disadvantage of this process is that toxic H₂S is produced.

DE 3046516 describes a single step process for making liquid polysulfides by first reacting NaSH, Bis-(2-chloroethoxy)-methane and 1,2,3 trichloropropane and thereafter introducing air (O₂) to thereby obtain a polysulfide. Polysulfides which are obtained according to the described procedure do not reach the required Shore-A-hardness after curing. This method is therefore unsuitable for production of polysulfide polymers on industrial scale.

US 2919262 describes the reduction of the polysulfide chain length by exchange reaction of the disulfide groups of the polymer backbone with terminal mercaptan groups of a short chain bis-thiol compound. Advantages of this splitting concept (compared to the use of sodium dithionite or sodium hydrosulfide/sodium sulfite) is incorporation of the splitting agent in the polymer backbone and that no salt formation takes place which would require a washing step. However, a major challenge with this concept is lack of a cost-effective process for producing the bis-thiol compound.

The present inventors identified bis-thiol compounds of Formula (I), such as 1,7-dimercapto-3,5-dioxaheptane (DMDH) and 1,8-dimercapto-3,6-dioxaoctane (DMDO), as compounds of particular interest in the splitting of the polysulfide latex: 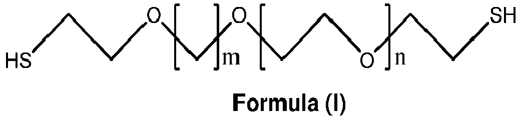 wherein m is 1 or 2 and n is an integer from 0 to 3.

Per the above, however, there remained a problem with obtaining such compounds in a safe, efficient, and cost-effective manner.

DE 737334 describes the synthesis of DMDH based on the splitting of a tetrasulfide polymer with sodium amalgam. This method leads to the formation of mercury as byproduct.

US 4122065 describes the preparation of DMDH by reaction of bis-(2-chloroethoxy)-methane and NaSH in the presence of a gaseous atmosphere of H₂S at excess pressure. This process requires a pressure resistant reactor and additional safety measures (H₂S is a highly toxic and flammable gas), which is associated with high costs. EP 0476339 discloses a similar process, albeit at lower pressure in the present of a phase transfer catalyst.

WO 2011/113774 discloses the production of bismercaptodiethers by reacting a polysulfide with a monothiol in the presence of a base.

There therefore remains a need for improvements in the process for producing liquid polysulfide polymers that avoid the above production safety, cost, and environmental issues. There also remains a need for a safe and cost-effective method for producing bis-thiol compounds of Formula (I) that are effective in the splitting of the polysulfide latex to generate liquid polysulfide polymers.

### Description of the Invention

The present inventors have now determined a safe and cost-effective method for producing bis-thiol compounds that are very effective at splitting high molecular weight polysulfide polymers and thus very efficient at producing the desired liquid polysulfide polymers. The process described below generates a mixture of bis-thiols of formula (I) and oligomers of formula (II) without requiring H₂S gas and without requiring a pressurized reactor.

It was determined that H₂S gas suppresses the formation of higher oligomers (Formula (II), below). Using an ambient atmosphere instead of an H₂S gas atmosphere generated a mixture of compounds, including the higher oligomers. An unexpected finding was that there was no need to separate the bis-thiol of formula (I) from the reaction mixture comprising higher oligomers (i.e., a purification/distillation step could be omitted, substantially improving the process efficiency), as the mixture (including higher oligomers of Formula (II)) generated by this process could be used to split the polysulfide with surprising efficiency. Furthermore, the splitting agent is incorporated into the resulting mercapto-terminated liquid polysulfide polymer, thereby improving overall yield and atom efficiency, and minimizing waste production. The process also removed the need for a separate salt washing step, thereby reducing process costs and further improving the environmental profile of the process.

Accordingly, in a first aspect, the present invention relates to a process for preparing a mixture comprising a) and b), wherein:
a) is one or more dimercapto compounds of Formula (I), 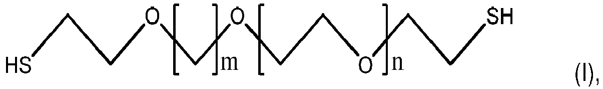 wherein m is 1 or 2 and n is an integer from 0 to 3;
b) is one or more oligomers of Formula (II), 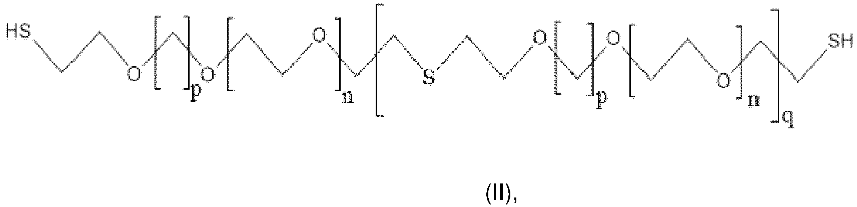 wherein p is 1 or 2, n is an integer from 0 to 3, and q is an integer from 1 to 3;
wherein the process comprises reacting a metal hydrosulfide with one or more organic dihalo compounds of Formula (III) 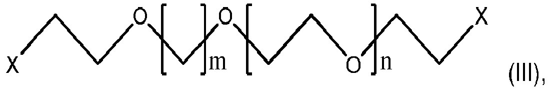 wherein m is 1 or 2; n is an integer from 0 to 3; X is a halogen; and wherein the reaction is performed under ambient conditions.

By performing the above reaction under ambient conditions, the need for a (pressurized) gaseous H₂S atmosphere is obviated, thereby substantially improving process safety. The term "ambient conditions" has its ordinary meaning, i.e., atmospheric pressure (the air pressure of the laboratory/factory/etc. environment, which is usually at or about standard pressure) and an air atmosphere (i.e., open to the environment and not placed under a specialized gas blanket, such as H₂S(g), N₂(g), etc.). The term "ambient conditions" thus excludes an overpressure of H₂S gas. It is acknowledged that small quantities of H₂S gas may be liberated during the course of the reaction, however that does not equate to performing the reaction under a gaseous H₂S atmosphere.

Preferably, the reaction is performed at elevated temperatures, preferably at a temperature of from about 70-110°C, preferably from about 80-105°C, most preferably from about 95-100°C.

Preferably, the reaction is performed in the absence of sodium hydroxide (NaOH).

Whilst the raw product (e.g., DMDH/oligomer mixture) could be purified by distillation, distillation of compounds of Formula (I) (e.g., DMDH) is expensive (e.g., DMDH boiling point 125 °C/18 mbar) and the higher oligomers would remain in distillation sump (waste). As noted above, however, an unexpected finding was that the higher oligomers produced by the above process contribute to the polysulfide splitting process, hence the isolation of the compound of Formula (I) (e.g., DMDH) is not necessary. When the mixture is used in the described splitting process, the polysulfide is preferably in the form of a latex, as this results in a much faster process, namely about 1 h with the latex, instead of about 24 hours with solid polysulfide.

Preferably, the metal hydrosulfide is an alkali metal hydrosulfide. In a most preferred embodiment, the alkali metal hydrosulfide is sodium hydrosulfide (NaSH).

Preferably, the molar ratio of the one or more organic dihalo compounds of Formula (III) to the metal sulfide in the reaction mixture is from about 1:2 to about 1:6, preferably from about 1:3 to about 1:5, and most preferably about 1:4.

In a preferred embodiment of the compounds of formula (III), m is 1. In a more preferred embodiment of the compounds of formula (III), m is 1 and n is 0.

Preferably, the organic dihalo compound is an organic dichloro compound, an organic dibromo compound, or an organic diiodo compound. Preferably still, the organic dihalo compound is an organic dichloro compound. In a preferred embodiment, the one or more organic dichloro compounds is a mixture of 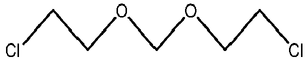 and

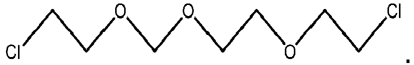

In another preferred embodiment, the one or more organic dichloro compounds primarily comprises or consists of bis-(2-chloroethoxy)-methane:

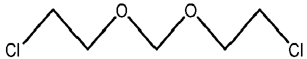

The production of such dihalo compounds is well known in the art. Pure bis-(2-chloroethoxy)-methane, for example, is commercially available. Technical grades of bis-(2-chloroethoxy)-methane can be obtained by reacting technical grade 2-chloroethanol with (para)formaldehyde. The technical grade bis-(2-chloroethoxy)-methane obtained from that reaction preferably contains >70wt.%, preferably still ≥80wt.% bis-(2-chloroethoxy)-methane, and preferably <30 wt.%, preferably still ≤20wt.% of higher chloroterminated polyethers. Using this easy-to-produce technical grade of bis-(2-chloroethoxy)-methane gives rise to a mixture of compounds of Formulae (I) and (II) (wherein the predominantly formed products are DMDH/DMDH oligomers) that have proven to be particularly suitable for splitting the high molecular weight polysulfide.

The above process for producing the mixture comprising a) and b) may optionally be carried out in the presence of surfactants. Surfactants are not essential for the above reaction, however using a surfactant can improve the ease of recovery of the oligomer mixture by phase separation. Anionic and/or non-ionic surfactants are preferred. Preferred anionic surfactants include, but are not limited to, anionic sulfonated or phosphorylated surfactants, preferably anionic sulfonated surfactants. Non-limiting examples of useful anionic sulfonated detergents in that respect is Nekal BX (sodium 3,6-dibutylnaphthalene-1-sulfonate), sodium dodecyl benzene sulfonate, and/or calcium dodecyl benzene sulfonate. Preferred non-ionic surfactants include, but are not limited to, polyalkylene oxides (including, but not limited to, polyethyleneglycols and alkylene oxide copolymers/terpolymers/etc., such as ethylene oxide-propylene oxide block copolymers) and alkoxylated (preferably ethoxylated) fatty alcohols (wherein the fatty alcohols, preferably primary C₈-C₃₀ fatty alcohols, may be branched or linear, saturated or unsaturated, substituted or unsubstituted, and may contain more than one alcohol moiety). Specific examples of suitable commercially available non-ionic surfactants include, but are not limited to, Emulan^{®} TO 3070 (an ethoxylated C₁₃-oxo alcohol with an average of 30 ethylene oxide units), PE 6200 (an ethylene oxide-propylene oxide block copolymer), the Lutensol^{®} range (alcohol ethoxylates and alkoxylates), and the Disponil^{®} range of nonionic surfactants.

A phase transfer catalyst (e.g., amines) may be used in the reaction, but is not required. Preferably, no amine phase transfer catalyst is used in the reaction, as such catalysts may provide residual amounts of toxic amines which, for safety reasons, are preferably to be avoided.

Thus, in a preferred embodiment, the process for preparing a mixture comprising components a) and b) described above comprises the steps of:
i) heating a solution of a metal hydrosulfide, preferably an alkali metal hydrosulfide, and optionally a surfactant, to a temperature of from about 70-110°C, preferably from about 80-105°C, most preferably from about 95-100°C,
ii) adding one or more organic dihalo compounds of Formula (III) to the solution of step i.,
iii) maintaining the reaction temperature for about 6-24 hours, preferably about 10-15 hours, more preferably about 12 hours and
iv) separating the mixture from the mother liquor, optionally washing the mixture with water.

A second aspect of the present disclosure relates to a mixture comprising compounds of formula (I) and formula (II) obtainable by the process described above. Preferably, the compounds of formula (I) and formula (II) constitute >70 wt.%, preferably >80 wt.%, preferably >85 wt.%, preferably >90 wt.%, and more preferably > 95 wt.% of the mixture obtained by the process described above (i.e., the total wt.% of the compounds of formulae (I) and (II) relative to the total weight of the mixture is preferably >70 wt.%, preferably >80 wt.%, preferably >85 wt.%, preferably >90 wt.%, and more preferably > 95 wt.%). Preferred mixtures include mixtures of DMDH/DMDH oligomers or mixtures of DMDO/DMDO oligomers. The most preferred mixtures comprise DMDH/DMDH oligomers, which are preferably obtained from pure or technical grade bis-(2-chloroethoxy)-methane using the methods described above. It has been found that the mixtures comprising DMDH/DMDH oligomers obtained by the above process are particularly suitable for the preparation of liquid polysulfide polymers.

A third aspect of the present disclosure relates to a process for preparing mercapto-terminated liquid polysulfide polymers. The process comprises reacting a high molecular weight polysulfide polymer, preferably in latex form, with the mixture comprising compounds of formula (I) and formula (II) obtainable by the process described above.

Producing a high molecular weight polysulfide polymer, particularly in latex form, is well known in the art and forms part of the common general knowledge in the field. In that respect, a "high molecular weight polysulfide polymer" generally relates to polysulfides having a weight average molecular weight of >50,000 g/mol, such as about 100,000-1,000,000 g/mol, preferably about 300,000-800,000 g/mol, more preferably about 400,000-600,000 g/mol. The weight average molecular weight may be determined by gel permeation chromatography (GPC).

Preferably, the splitting reaction takes place at elevated temperatures, preferably at a temperature of from about 70-110°C, preferably from about 80-105°C, preferably from about 90-100°C, most preferably at about 95°C.

The liquid polysulfide polymers produced by the splitting reaction preferably have a weight average molecular weight of less than about 30,000 g/mol, preferably less than about 15,000 g/mol, more preferably less than about 10,000 g/mol, and most preferably about 1000-5000 g/mol. Again, the weight average molecular weight may be determined by gel permeation chromatography (GPC).

The following examples provide a detailed method for working the invention. These worked examples are exemplary in nature and not intended to be limitative.

### Examples

### Synthesis of bis-(2-chloroethoxy)-methane (technical grade, 80% purity)

A mixture of 4 moles paraformaldehyde, 10 moles ethylenechlorohydrin (technical grade) and 5,4 g 37% HCI is heated to 60°C while stirring, until solubilization of the paraformaldehyde occurred. The reaction mixture is then subjected to an azeotropic distillation in two steps under reduced pressure (120 mbar/54°C and 20 mbar/94°C head temperature) to remove the reaction water with excess ethylenechlorohydrin. 680 g Bis-(2-chloroethoxy)-methane (technical grade, 80% purity) is formed.

### Synthesis of oligomer mixture containing 1,7-dimercapto-3,5-dioxaheptane (DMDH)

A solution of 822 mL NaHS 8,8 mol/L in 380 mL water and 0,5 g Nekal BX (surfactant) is heated to 95°C and 317 g of bis-(2-chloroethoxy)-methane (technical grade, 80% purity) is added over 1 h. After adding the dihalide the reaction mixture is allowed to stir at 95-100°C for 12h. After this time the mother liquor (i.e. the aqueous phase) is separated from the organic phase (i.e. the product), and the product is washed with 600 mL water. The resulting product (called "DMDH/oligomer mixture") is a mixture of DMDH and higher oligomers.

### Reduction with DMDH/oligomer mixture

A suspension of high molecular weight polysulfide latex in water, obtained by the reaction of 322 g of Bis-(2-chloroethoxy)-methane with 544 mL sodium polysulfide (x-value 2,5; 3,27 mol/L), is heated to 95°C and then treated with 60 mL NaHSO₃ 39%, 24,1 g NaOH 50% and 45 g of DMDH oligomer product. The reaction mixture is allowed to stir for 1 h at 95°C. Afterwards the mother liquor is separated, treated with acetic acid, washed and dried. The resulting polymer (365 g) has a viscosity of 11,8 Pa*s.

### Reduction with sodium dithionite (Comparative Example)

A suspension of high molecular weight polysulfide latex in water, obtained by the reaction of 322 g of Bis-(2-chloroethoxy)-methane with 544 mL sodium polysulfide (x-value 2,5; 3,27 mol/L), is heated to 98°C and then treated with 27,8 g Na₂S₂O₄, 60 mL NaHSO₃ 39% and 47,1 g NaOH 50%. The reaction mixture is allowed to stir for 1 h at 98°C. Afterwards the mother liquor is separated, the salts are washed out, the latex is then treated with acetic acid, washed and dried. The resulting polymer (320 g) has a viscosity of 10 Pa*s.

The DMDH/oligomer mixture obtained by the method of the present disclosure generated a higher yield of liquid polymer vis-à-vis sodium dithionite and did not require a salt washing step. The resulting liquid polymer was also of comparable quality to that produced by conventional methods (reduction with sodium dithionite). The present disclosure therefore provides a method for producing liquid polysulfides that is higher yielding, more atom efficient, more environmentally friendly, and safer to perform than conventional methods that use H₂S gas or sodium dithionite.

While at least one exemplary embodiment has been presented in the foregoing detailed description of the disclosure, it should be appreciated that a vast number of variations exist. It should also be appreciated that the exemplary embodiment or exemplary embodiments are only examples, and are not intended to limit the scope, applicability, or configuration of the disclosure in any way. Rather, the foregoing detailed description will provide those skilled in the art with a convenient road map for implementing an exemplary embodiment of the disclosure. It being understood that various changes may be made in the function and arrangement of elements described in an exemplary embodiment without departing from the scope of the disclosure as set forth in the appended claims.

## Claims

1. A process for preparing a mixture comprising a) and b), wherein:
a) is one or more dimercapto compounds of Formula (I), 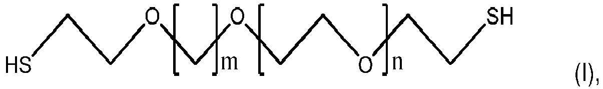 wherein m is 1 or 2 and n is an integer from 0 to 3; and
b) is one or more oligomers of Formula (II), 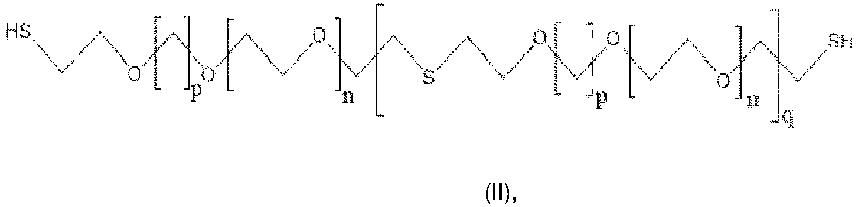 wherein p is 1 or 2, n is an integer from 0 to 3, and q is an integer from 1 to 3;
wherein the process comprises reacting a metal hydrosulfide with one or more organic dihalo compounds of Formula (III), 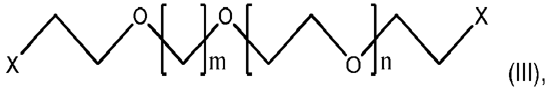 wherein m is 1 or 2; n is an integer from 0 to 3; X is a halogen; and wherein the reaction is performed under ambient conditions.

2. The process according to claim 1, wherein m is 1.

3. The process according to claims 1 or 2, wherein X is a chlorine radical.

4. The process according to claim 3, wherein the one or more organic dihalo compounds of Formula (III) is a mixture of organic dihalo compounds of Formula (III) comprising 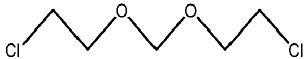 and

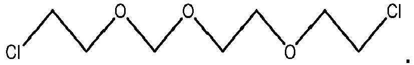

5. The process according to claim 3, wherein the one or more organic dihalo compounds of Formula (III) comprises or consists of

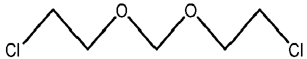

6. The process according to any one of claims 1 to 5, wherein the metal hydrosulfide is an alkali metal hydrosulfide.

7. The process according to claim 6, wherein the alkali metal hydrosulfide is sodium hydrosulfide (NaSH).

8. A mixture comprising compounds of formula (I) and formula (II) obtainable by the process of any one of claims 1 to 7.

9. The mixture of claim 8, wherein the compound of formula (I) is 1,7-dimercapto-3,5-dioxaheptane (DMDH) and the compounds of formula (II) are oligomers of DMDH.

10. A method for preparing mercapto-terminated liquid polysulfide polymers comprising reacting a high molecular weight polysulfide polymer with the mixture of claims 8 or 9.

11. The method of claim 10, wherein the high molecular weight polysulfide polymer is in latex form.

12. A mercapto-terminated liquid polysulfide polymer obtainable by the method of claims 10 or 11.

13. Use of the mixture of claims 8 or 9 for splitting a high molecular weight polysulfide polymer.

14. The use of claim 13, wherein the high molecular weight polysulfide polymer is in latex form.

15. The method of claims 10-11 or the use of claims 13-14, wherein the high molecular weight polysulfide polymer has a weight average molecular weight of >50,000 g/mol.
